# FASCICULE DE BREVET EUROPEEN

(11) **EP 0 684 851 B1**
(45) Date de publication et mention de la délivrance du brevet: **29.04.1998**
(21) Numéro de dépôt: 94906947.0
(22) Date de dépôt: 15.02.1994
(51) Int. Cl.: A61M 15/00

(54) **DISPOSITIF PORTATIF DE PULVERISATION A ACTIONNEMENT DECLENCHE PAR L'INHALATION**
TRAGBARE, DURCH DIE INHALATION BETÄTIGTE , ZERSTÄUBERVORRICHTUNG
HAND-HELD INHALATION-ACTUATED SPRAY DEVICE

(30) Priorité: 16.02.1993 FR 9301734
(43) Date de publication de la demande: 06.12.1995
(73) Titulaire: VALOIS S.A., 27110 Le Neubourg (FR)
(72) Inventeur: BRUNA, Pascal, F-76000 Rouen (FR); BUFFET, Jacques, F-93250 Villemomble (FR)
(74) Mandataire: Pinguet, André
(86) Numéro de dépôt international: FR9400166
(87) Numéro de publication internationale: WO9419040

(56) Documents cités:
- EP-A- 0 186 280
- EP-A- 0 414 536
- WO-A-92/07599
- WO-A-92/11054
- US-A- 4 109 656
- US-A- 4 198 626
- US-A- 5 097 255

## Description

La présente invention concerne un dispositif portatif de pulvérisation à actionnement déclenché par l'inhalation, et plus particulièrement un pulvérisateur au moyen duquel on pulvérise une dose de médicament dans les voies respiratoires d'un patient, en synchronisme avec l'aspiration dudit patient.

On connait de nombreux dispositifs mécaniques de ce type, comportant :
- une réserve d'une substance à pulvériser,
- un dispositif de distribution ayant un organe d'actionnement mobile entre une position de repos et une position d'actionnement, ledit dispositif de distribution émettant une dose mesurée de ladite substance lorsque l'organe d'actionnement est déplacé de sa position de repos à sa position d'actionnement, ledit organe d'actionnement étant rappelé élastiquement vers sa position de repos, le dispositif de distribution ayant en outre une sortie pour émettre ladite substance,
- un conduit d'inhalation dans lequel un patient peut aspirer de l'air, et qui communique avec ladite sortie de ladit substance,
- des moyens de sollicitation de l'organe d'actionnement pour solliciter ledit organe d'actionnement vers sa position d'actionnement,
- un organe de verrou mobile entre une position de verrouillage, où il bloque l'action desdits moyens de sollicitation, et une position de libération, où il ne bloque plus l'action desdits moyens de sollicitation,
- des moyens de déblocage pour déplacer ledit organe de verrou vers sa position de libération, lors d'une aspiration dans le conduit d'inhalation.

Dans ces dispositifs purement mécaniques, les moyens pour déverrouiller le mécanisme de verrouillage comportent généralement une ailette déplaçable par l'inhalation du patient. Des exemples de tels dispositifs sont donnés dans les documents : US-A-3 456 645, US-A-3 456 646, US-A-3 598 294, US-A-3 605 738, US-A-3 636 949, US-A-3 732 864, US-A-3 789 843, US-A-3 814 297, GB-A-1 392 192, CH-A-511 063, FR-A-2 615 106, FR-A-2 658 081, EP-A-0 147 028 (et brevet correspondant US-A-4 664 107), EP-A-0 414 536, EP-A-0 428 380, EP-A-0 045 419, DE-A-30 40641, DE-A-40 15367, et DE-U-89 12098.

Ces dispositifs purement mécaniques présentent l'inconvénient que l'énergie nécessaire pour faire passer le mécanisme de verrouillage de son état verrouillé à son état déverrouillé, est fournie uniquement par l'aspiration du patient, donc ils impliquent une aspiration assez forte du patient. Dans ces systèmes, on peut très difficilement minimiser l'énergie que doit fournir le patient pour faire passer le système de verrouillage de l'état verrouillé à l'état déverrouillé, car cela pourrait entraîner des déclenchements intempestifs du dispositif. Or ces dispositifs doivent pouvoir être utilisés même par des personnes ayant une faible capacité d'aspiration, tels que des enfants, des personnes agées, des asthmathiques, des personnes affaiblies, etc..

De plus, dans les dispositifs de l'art antérieur, le niveau d'aspiration nécessaire pour déclencher la pulvérisation dépend des frottements entre le pièces mécaniques, dont on sait qu'ils sont très difficiles à maîtriser, et qu'ils varient avec l'usure du matériau. Ces systèmes sont donc peu précis quant au niveau d'aspiration qui déclenche la pulvérisation.

La présente invention a pour but de supprimer ces inconvénients.

La présente invention a donc pour objet un dispositif du type mentionné ci-dessus comportant:
- un actionneur électrique pour déplacer ledit organe de verrou,
- un circuit électronique de commande connecté audit actionneur électrique pour le commander,
- une source d'énergie pour alimenter ledit circuit électronique de commande, et
- un capteur d'aspiration pour envoyer audit circuit électronique de commande un signal d'aspiration, lorsqu'il détecte une aspiration dans le conduit d'inhalation, le circuit électronique de commande commandant alors l'actionneur électrique pour déplacer l'organe de verrou dans sa position de libération,
et caractérisé en ce qu'il comporte en outre un capteur de position pour détecter une orientation correcte dudit dispositif, dans lequel ledit capteur de position est connecté audit circuit électronique de commande, et ledit circuit électronique de commande est adapté à ne déclencher l'actionnement du dispositif que si ledit capteur de position détecte ladite orientation correcte du dispositif.

Avantageusement, ledit capteur d'aspiration mesure une dépression dans ledit conduit d'inhalation par rapport à la pression atmosphérique et envoie au circuit électronique de commande un signal représentatif de la dépression.

Un problème technique rencontré dans les pulvérisateurs déclenchés par l'inhalation, est que la dépression créée dans le conduit d'inhalation par l'aspiration du patient, varie selon les patients. Donc pour un patient ayant une faible force d'aspiration, la pulvérisation peut être déclenchée trop tard dans le cycle respiratoire, ou même ne pas être déclenchée.

Selon une forme de réalisation, de l'invention, ce problème est résolu en ce que ledit capteur de position comporte des moyens pour détecter une position particulière du dispositif et faire fonctionner ledit circuit électronique de commande en mode d'étalonnage, auquel cas le dispositif n'est pas actionné lorsque le patient aspire dans le conduit d'inhalation, mais ledit circuit électronique de commande détermine alors la dépression maximale créée dans le conduit d'inhalation au cours de l'aspiration, et calcule et mémorise une dépression de déclenchement en fonction de ladite dépression maximale, le dispositif étant ultérieurement actionné lorsqu'une dépression supérieure ou égale à ladite dépression de déclenchement est détectée dans le conduit d'inhalation et lorsque le circuit électronique de commande ne fonctionne plus en mode d'étalonnage. Dans ce cas, le dispositif de l'invention peut comporter en outre des moyens d'affichage connectés au circuit électronique de commande pour afficher une valeur de débit d'air inhalé par le patient en fonction de la dépression mesurée dans le conduit d'inhalation.

Un autre problème est de garantir une bonne homogénéité de la substance contenue dans le réservoir de l'appareil, lorsque cette substance est un mélange ou une suspension. Selon une forme de réalisation de l'invention, ce problème est résolu par un dispositif comportant un capteur d'agitation pour détecter une agitation de ladite réserve, dans lequel ledit capteur d'agitation est connecté audit circuit électronique de commande, et ledit circuit électronique de commande est adapté à ne pas déclencher l'actionnement du dispositif si ladite réserve n'a pas été agitée au cours d'une deuxième durée prédéterminé précédant l'inhalation du patient.

Lorsque le dispositif de distribution est une pompe ou une valve aérosol qui pulvérise un liquide ou semi-liquide, il est nécessaire que le dispositif soit correctement orienté au moment où l'organe d'actionnement revient dans sa position de repos, pour ne pas désarmorcer la pompe ou la valve.

Selon une forme de réalisation, la substance à pulvériser est liquide ou semi-liquide, le dispositif de distribution est une pompe ou une valve aérosol, le circuit électronique de commande est connecté à des moyens pour détecter le déplacement de l'organe d'actionnement de sa position d'actionnement à sa position de repos, le circuit électronique de commande est connecté à des moyens avertisseurs et ledit circuit électronique de commande est adapté à commander les moyens avertisseurs pour prévenir le patient que la dose suivante de ladite substance qui sera délivrée par le dispositif, sera incomplète, dans le cas où ledit déplacement de l'organe d'actionnement est détecté lorsque le dispositif n'est pas dans son orientation correcte. Avantageusement, le dispositif comporte des moyens pour actionner manuellement la pompe ou la valve et dans lequel lesdits moyens avertisseurs indiquent au patient d'actionner manuellement une fois la pompe ou la valve. Eventuellement, le dispositif comporte des moyens pour compter les doses de substance émises, et dans lequel l'émission de ladite dose suivante n'est pas comptée. Ces dispositions sont particulièrement valables pour une valve doseuse ou éventuellement une pompe fonctionnant en position inversée où un seul actionnement suffit à coup sûr à réamorcer la valve ou la pompe.

Avantageusement, ledit capteur de position comporte une colonne creuse qui est orientée verticalement lorsque le dispositif est dans sa position correcte, ladite colonne creuse ayant une extrémité inférieure dotée de deux contacts électriques et ladite colonne creuse contenant une goutte de mercure qui mouille les deux contacts électriques lorsque le dispositif est dans sa position correcte. Avantageusement, ledit capteur de position sert aussi de capteur d'agitation, et ledit circuit électronique de commande est adapté à ne pas déclencher l'actionnement du dispositif si ladite réserve n'a pas été agitée au cours d'une deuxième durée prédéterminé précédant l'inhalation du patient.

Selon une forme de réalisation, ledit actionneur électrique comporte un électro-aimant, l'organe de verrou étant en liaison mécanique avec une culasse mobile sensible aux champs magnétiques, ladite culasse mobile étant disposée face audit électro-aimant, ledit électro-aimant comporte un moyen à aimantation permanente, qui maintient ladite culasse dans une position d'attente, ldedit électro-aimant comporte en outre une bobine, et le circuit électronique de commande est adapté à alimenter ladite bobine par un courant de polarisation et d'intensité adaptées à faire passer ladite culasse mobile dans une position d'actionnement, ladite culasse mobile entraînant ainsi l'organe de verrou de sa position de verrouillage à sa position de libération.

De préférence, la culasse mobile comporte au moins un ergot coopérant avec ledit organe de verrou, ladite culasse mobile étant montée rotative sur un axe et ledit organe de verrou étant monté rotatif sur un axe, lesdits axes étant solidaires d'une platine, l'ergot entraînant l'organe de verrou en rotation, de sa position de verrouillage où il coopère avec une goupille solidaire de la réserve de produit, à sa position de libération.

Selon un mode de réalisation, l'électro-aimant est quadripolaire et la culasse mobile est maintenue en équilibre instable par opposition de pôles identiques dans sa position de verrouillage, un signal du circuit électronique de commande diminuant la force d'attraction de l'électro-aimant, de sorte que la répulsion magnétique créée par l'opposition de pôles entraîne ladite culasse en rotation vers sa position d'actionnement.

Avantageusement, un second organe de verrou est monté rotatif sur un axe solidaire de la platine sensiblement identique à l'organe de verrou et disposé symétriquement audit premier organe de verrou par rapport à l'axe supportant la culasse mobile, ledit second organe de verrou coopérant avec une seconde goupille solidaire de la réserve de produit et étant entraîné dans sa position de libération par un second ergot disposé symétriquement au premier ergot sur la culasse mobile, ledit second organe de verrou empêchant le dispositif de distribution de retourner dans sa position de repos.

Avantageusement, la culasse mobile comporte un bras s'étendant en dehors du boîtier pour actionner ladite culasse mobile, et donc déplacer l'organe de verrou dans sa position de libération, indépendamment d'une aspiration dans le conduit d'inhalation.

Selon une forme de réalisation, le dispositif de distribution est solidaire de la réserve de substance à pulvériser, la position d'actionnement de l'organe d'actionnement est telle que ledit organe d'actionnement est rapproché de ladite réserve pour passer de la position de repos à la position d'actionnement, le dispositif portatif de pulvérisation comporte un boîtier solidaire du conduit d'inhalation et de l'organe d'actionnement, le dispositif portatif de pulvérisation comporte en outre un capot articulé sur ledit boîtier et mobile entre une position fermée, où il ferme le conduit d'inhalation, et une position ouverte où il ouvre le conduit d'inhalation, le dispositif portatif de pulvérisation comporte en outre un ressort de compression adapté à solliciter la réserve vers l'organe d'actionnement pour déplacer ledit organe d'actionnement dans sa position d'actionnement, ledit ressort de compression ayant une première extrémité qui agit sur la réserve et une deuxième extrémité qui agit sur un organe d'appui, le capot comporte une partie formant levier qui appuie sur l'organe d'appui lorsque le capot est déplacé dans sa position ouverte, en comprimant le ressort de compression et ledit organe de verrou bloque la réserve lorsqu'il est dans sa position de verrouillage, la première extrémité du ressort de compression agit sur un organe intermédiaire fixé à ladite réserve et ledit organe intermédiaire comporte un logement pour recevoir ledit organe de verrou lorsqu'il est dans sa position de verrouillage, ledit organe intermédiaire collaborant alors avec l'organe de verrou pour bloquer la réserve.

Dans un cas particulier, l'organe d'actionnement est déplaçable relativement au dispositif de distribution parallèlement à un axe, le capot est monté rotatif autour de deux pivots et les pivots sont montés chacun dans un chemin de guidage allongé parallèle à l'axe, les pivots étant ainsi déplaçables axialement dans leurs chemins de guidage contre la force du ressort de compression, et le capot comporte en outre deux saillies latérales dirigées vers l'extérieur, qui collaborent chacune avec une surface saillante du boîtier dirigée vers l'intérieur, pour obliger les pivots à se déplacer dans leurs chemins de guidage en comprimant le ressort de compression, lorsque le capot passe de sa position fermée à sa position ouverte. Avantageusement, pour permettre le remplacement de la réserve de substance et de son dispositif de distribution les deux chemins de guidage comportent deux extrémités axiales et présentent chacun une ouverture disposée au même niveau sur les deux chemins de guidage, à une position intermédiaire entre les deux extrémités axiales de chemins de guidage, pour permettre la sortie des ergots hors des chemins de guidage lorsqu'on appuie sur le capot parallèlement à l'axe et qu'en même temps on sollicite le capot perpendiculairement à l'axe dans la direction desdites ouvertures, le capot étant ainsi démontable du boîtier, le boîtier comportant une ouverture de chargement de taille permettant le passage de la réserve de substance avec son dispositif de distribution, l'organe d'actionnement du dispositif de distribution étant fixé de façon amovible audit boîtier, et le boîtier comporte des moyens pour déplacer l'organe de verrou dans sa position de libération, indépendamment d'une aspiration dans le conduit d'inhalation. Avantageusement ladite partie formant levier du capot présente une surface d'appui en contact avec l'organe d'appui et ladite surface d'appui comporte deux méplats s'appuyant sur ledit organe d'appui respectivement dans la position fermée et dans la position ouverte du capot.

Selon une forme de réalisation de l'invention, le boîtier comporte une ouverture de taille adaptée à permettre le passage de la réserve avec son dispositif de distribution pour la rentrer dans le boîtier ou la sortir du boîtier et ledit boîtier comporte en outre un organe de support qui est solidaire de l'organe d'actionnement et qui est monté amovible dans ladite ouverture.

Selon une forme de réalisation, le dispositif de distribution comporte en outre un capteur pour détecter la présence de la réserve et/ou du dispositif de distribution, ledit capteur étant connecté au circuit électronique de commande pour empêcher le fonctionnement du dispositif en cas d'absence de ladite réserve.

Selon une forme de réalisation, le dispositif comporte en outre un capteur pour détecter la présence de la réserve et/ou du dispositif de distribution, ledit capteur étant connecté au circuit électronique de commande, ledit circuit électronique de commande est adapté à compter le nombre de réserves qui ont été mises en place dans ledit dispositif en fonction des indications dudit capteur de détection de présence. Avantageusement, ledit circuit électronique de commande est adapté à empêcher tout actionnement du dispositif si ledit nombre de réserves qui ont été mises en place dans le dispositif dépasse un nombre prédéterminée.

D'autres caractéristiques et avantages de l'invention apparaîtront au cours de la description détaillée suivante d'une forme de réalisation particulière de l'invention, donnée à titre d'exemple non limitatif, en regard des dessins joints.

Sur les dessins :
- la figure 1 est une vue en perspective d'un dispositif selon une forme de réalisation de l'invention, en position fermée,
- la figure 2 est une vue en perspective du dispositif de la figure 1, en position ouverte,
- la figure 3 est une vue en perspective du capot du dispositif de la figure 1,
- la figure 4 est une en vue en coupe verticale du dispositif de la figure 1, en position fermée,
- la figure 4a est une vue de détail de la figure 4,
- la figure 4b est une autre vue de détail de la figure 4,
- la figure 5 est en vue en coupe du boîtier du dispositif de la figure 4,
- la figure 6 est une vue en perspective représentant un détail du boîtier de la figure 5,
- la figure 7 est une vue en coupe similaire à la figure 4, en position ouverte, avant inhalation,
- la figure 8 est une vue similaire à la figure 7, après inhalation,
- la figure 9 est une vue schématique en coupe représentant le capteur de pression du dispositif de la figure 1,
- la figure 10 est un schéma-bloc du circuit électronique du dispositif de la, figure 1,
- la figure 11 est une vue en coupe transversale du mécanisme de verrouillage selon l'invention,
- la figure 12 est une vue en plan du mécanisme de verrouillage selon un mode de réalisation de l'invention,
- la figure 13 est une vue du dispositif électromagnétique du mécanisme de verrouillage de la figure 12,
- la figure 14 est une vue en coupe verticale d'une variante du dispositif de la figure 1, et
- la figure 15 est une vue partielle de dessous du dispositif de la figure 17, selon la direction XVIII.

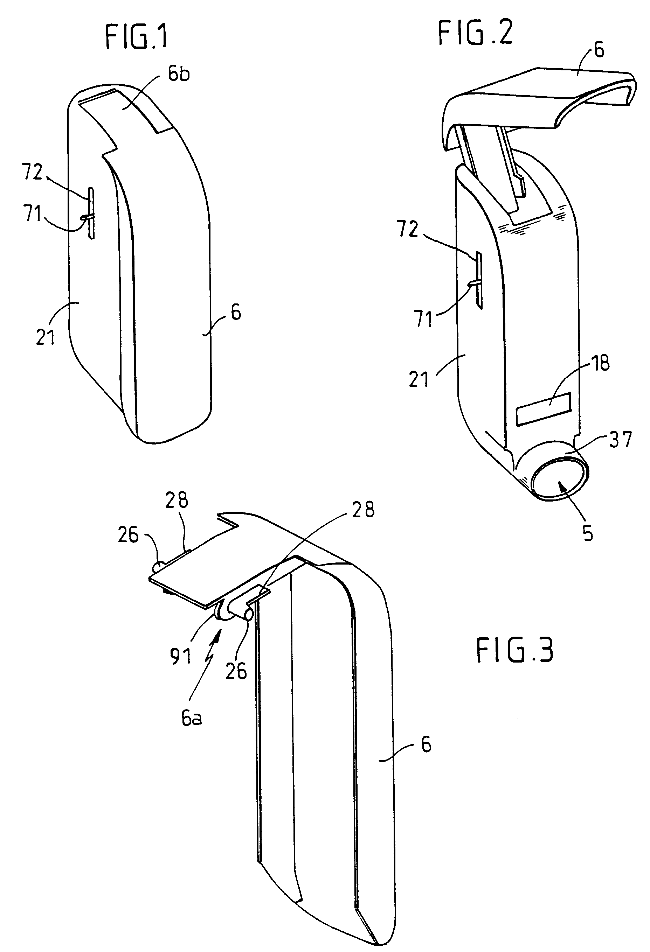

Dans la forme de réalisation représentée sur les dessins, le dispositif de l'invention comporte un boîtier 21 doté d'un capot 6, comme on peut le voir sur la figures 1 et 2. Le boîtier 21 comporte un embout 37, bucal ou nasal, qui délimite un conduit d'inhalation 5. Le boîtier 21 comporte en outre un écran 18, qui permet d'afficher différents messages au patient qui utilise l'appareil. Le capot 6 est monté rotatif sur le boîtier 21, de façon à être mobile entre une position fermé (figure 1), où il recouvre l'embout 37, et une position ouverte (figure 2) où il découvre l'embout 37.

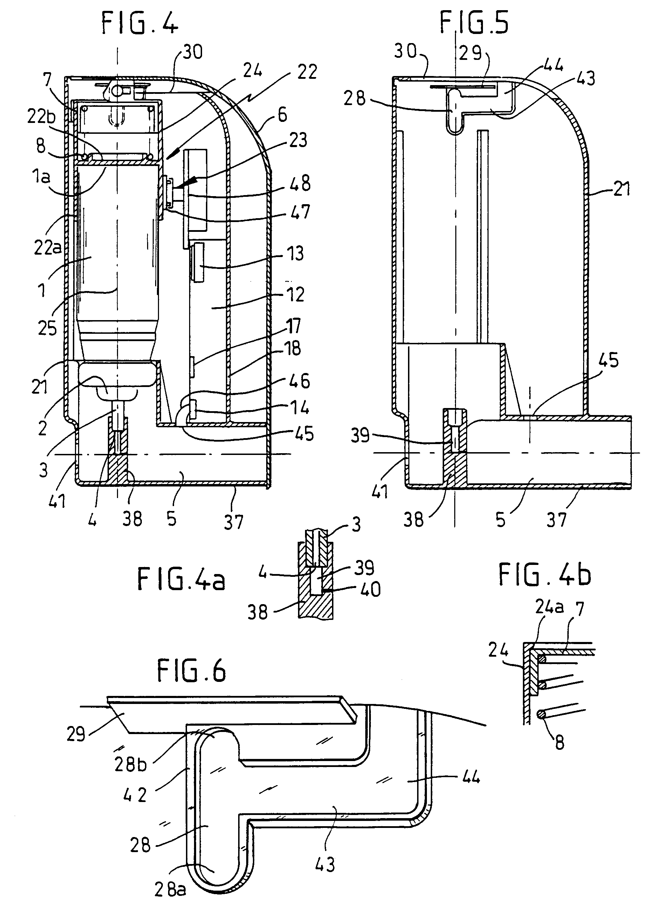

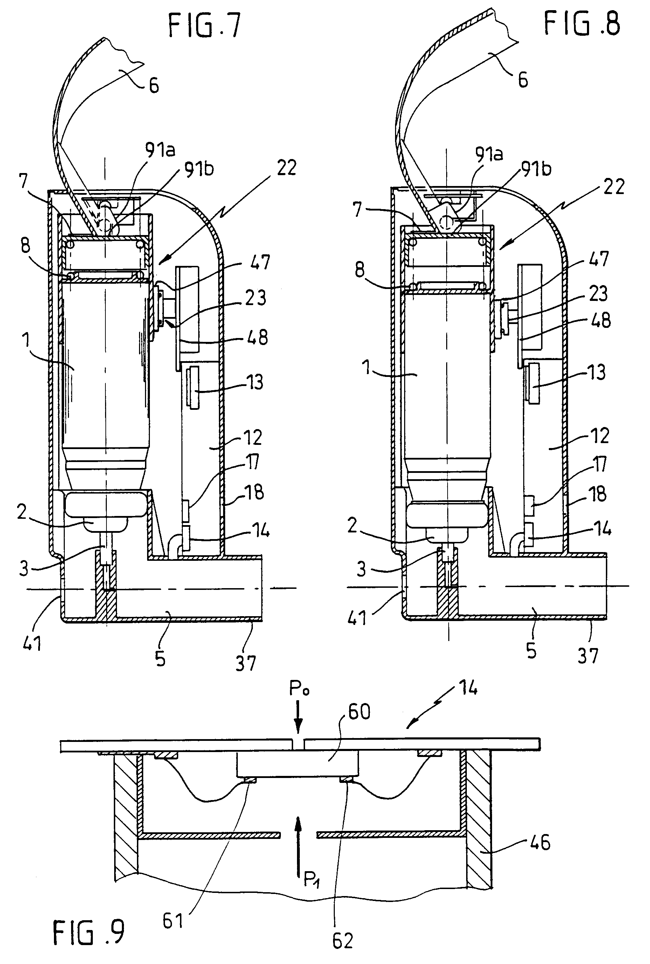

Le dispositif des figures 1 et 2 est visible avec plus de détails sur la figure 4. Dans l'exemple représenté, le dispositif comporte un pulvérisateur aérosol qui comprend un réservoir 1 qui contient une substance active (médicament) mélangée à un gaz propulseur liquéfié, une valve doseuse 2 sertie sur le réservoir 1, et une tige d'actionnement creuse 3, dotée d'une extrémité de sortie 4. Dans l'exemple particulier représenté, la valve doseuse 2 est du type fonctionnant en position inversée, mais la valve doseuse 2 pourrait être aussi du type fonctionnant en position droite, ou être remplacée par une pompe ou un autre moyen de distribution. La tige d'actionnement 3 est mobile entre une position de repos, représentée sur la figure 4, et une position d'actionnement représentée sur la figure 8, où la tige d'actionnement 3 est enfoncée dans la valve doseuse 2. Lorsque la tige d'actionnement 3 est déplacée de sa position de repos vers sa position d'actionnement, une dose mesurée de substance active est émise par l'extrémité de sortie 4 de la tige d'actionnement. De façon classique, la tige d'actionnement 3 est rappelée élastiquement vers sa position de repos, par un ressort de rappel interne (non représenté).

Comme on peut le voir sur les figures 4 et 4a, l'extrémité de sortie 4 de la tige d'actionnement 3 est emboîtée dans un alésage borgne 39 d'un plot 38 disposé dans le conduit d'inhalation 5. L'alésage borgne 39 débouche dans le conduit d'inhalation 5 par un orifice latéral 40 de faible section, qui est dirigé vers l'embout 37.

Le conduit d'inhalation comporte en outre un orifice d'entrée d'air 41, qui permet l'établissement d'un flux d'air lorsqu'un patient aspire dans le conduit d'inhalation 5 par l'embout 37.

Dans l'exemple représenté, le réservoir 1, la valve doseuse 2 et la tige d'actionnement 3 sont de symétrie de révolution autour d'un axe 25. Le réservoir 1 comporte un fond la, sur lequel est emboîtée une douille 22. La douille 22 comporte une paroi latérale 22a cylindrique, et un fond 22b qui recouvre le fond la du réservoir 1. A partir du fond 22b de la douille 22, une paroi latérale cylindrique de guidage 24, symétrique de révolution autour de l'axe 25, s'étend sur une certaine distance en direction opposée du réservoir 1. Un piston 7 est monté axialement coulissant dans la paroi de guidage 24, et un ressort de compression 8 est disposé entre le fond 22b et le piston 7. De préférence, le piston 7 coulisse sans étanchéité à l'intérieur de la paroi latérale de guidage 24 pour éviter que les déplacements axiaux du piston 7 ne créent des surpression ou des dépressions d'air dans l'espace comprise entre le piston 7 et le fond 22b de la douille 22.

La douille 22 est adaptée à coulisser dans le boîtier parallèlement à l'axe 25. Elle est guidée dans son mouvement de coulissement par trois nervures de guidage 73, 74, 75 du boîtier 21, qui sont orientées parallèlement à l'axe 25 (voir figure 16). En outre, la paroi latérale 22a de la douille 22 comporte elle-aussi deux nervures axiales 76, qui collaborent avec la nervure 75 pour fixer la position de la douille 22 en rotation autour de l'axe 25.

Comme représenté sur la figure 3, le capot 6 est monté rotatif sur le boîtier 21 au moyen de deux pivots 26. En outre, au voisinage des pivots 26, le capot 6 présente deux plaques saillantes 28, dirigées vers l'extérieur, qui sont disposées horizontalement lorsque le capot est dans la position fermée représentée sur la figure 4. En outre, le capot 6 comporte au voisinage de chaque pivot 26, une surface d'appui 91, qui est en contact avec le piston 7. Comme il sera vu ci-dessous la plaque saillante 28 forme avec la surface d'appui 91 un levier 6a qui permet de comprimer le ressort de compression 8.

En effet, comme on peut le voir sur les figures 5 et 6, le boîtier 21 comporte latéralement deux chemins de guidage 28 parallèles à l'axe 25, qui reçoivent chacun un pivot 26 du capot 6. Chaque chemin de guidage 28 est creusé dans une surépaisseur 42 de la paroi du boîtier 21, et s'étend entre deux extrémités axiales 28a, 28b. En outre pour des raisons qui seront expliquées en détail ci-dessous, un canal de démontage 43 est creusé lui-aussi dans la surépaisseur 42, perpendiculairement à l'axe 25, et le canal de démontage 43 débouche dans le chemin de guidage 28, à une position intermédiaire entre ses deux extrémités axiales 28a et 28b. En outre, le canal de démontage 43 communique avec un puits de sortie 44, disposé parallèlement à l'axe 25, et qui débouche en partie supérieure du boîtier 21.

En outre, le boîtier 21 comporte, au-dessus de chaque chemin de guidage 28, une plaque 29 qui saille intérieurement, et qui est disposée perpendiculaire à l'axe 25. Lorsque le capot 6 est basculé de sa position fermée à sa position ouverte, les plaques saillantes 28 du capot s'appuyent sous les plaques saillantes 29 du boîtier pour déplacer vers le bas les pivots 26, dans leur chemin de guidage 28. Au cours de ce mouvement, les surfaces d'appui 91 du capot 6 déplacent le piston 7 vers le bas, en comprimant le ressort 8.

Avantageusement, chaque surface d'appui 91 du capot 6 comporte deux méplats 91a, 91b, qui sont en appui contre le piston 7, respectivement lorsque le capot 6 est dans sa position fermée et dans sa position ouverte. Lorsque le ressort 8 est comprimé, comme représenté sur la figure 7, il tend à déplacer vers le bas de la douille 22 et le réservoir 1, et donc à déplacer la tige d'actionnement 3 vers sa position d'actionnement. Toutefois, la paroi latérale 22b de la douille 22 comporte un organe de butée 47 qui collabore avec un mécanisme de verrouillage 23 pour maintenir en place la douille 22 et le réservoir 1, tant qu'aucune aspiration n'est détectée dans le conduit d'inhalation 5. Le mécanisme de verrouillage 23 est fixé sur une platine 48, qui dans cet exemple est métallique, et qui est fixée à l'intérieur du boîtier 21 par tout moyen connu, par exemple par vissage, par clipsage, etc..

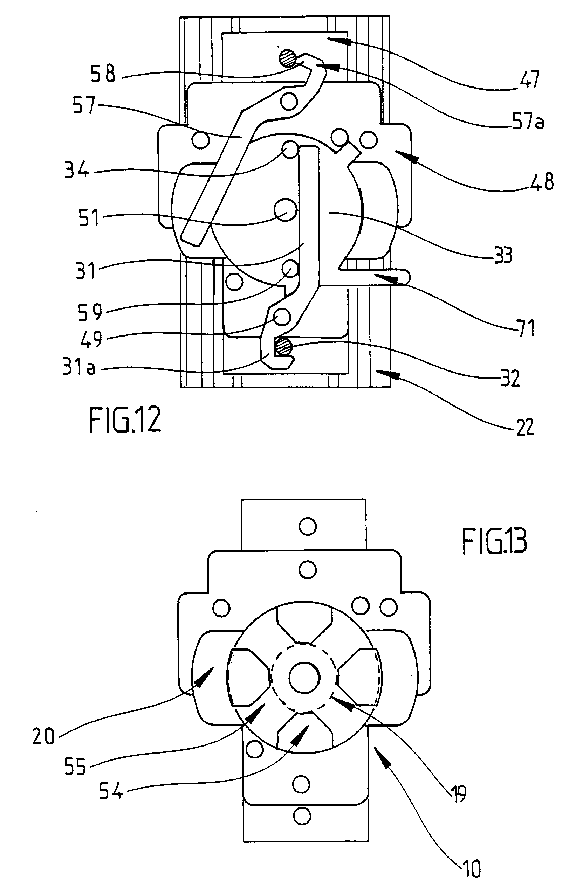

Le mécanisme de verrouillage est représenté plus en détail sur les figures 11 et 13. Il comporte au moins un organe de verrou, avantageusement un crochet de verrouillage 31, monté rotatif autour d'un axe 49 solidaire de la platine 48. Le crochet de verrouillage comporte une tête 31a, adaptée à s'engager sur une goupille 32 de l'organe de butée 47, pour bloquer la douille 22. Le crochet de verrouillage est ainsi mobile entre une position de verrouillage dans laquelle la tête 31a est engagée sur la goupille 32, et une position de libération dans laquelle la tête 31a n'interfère plus avec l'organe de butée 47, ce qui libère la douille 22.

Le crochet de verrouillage 31 est monté rotatif sur un axe 49 solidaire de la platine 48. Egalement solidaire de ladite platine 48 se trouve un axe 51 sur lequel est monté rotative une culasse mobile 33. Cette culasse comporte sur une surface un premier ergot 34 faisant saillie sur lequel vient s'appuyer l'extrémité du crochet de verrouillage 31 qui est opposé à la tête du crochet 31a. L'autre surface de la culasse 33 fait face à un électro-aimant 10. Avantageusement, cet électro-aimant 10 comporte un aimant quadripolaire 19 et une bobine 20, ledit aimant 19 comportant un plat stator 54 et des plaques de palier 55 qui détermine la polarisation dudit aimant.

De préférence, le mécanisme de verrouillage comporte en outre des moyens pour empêcher la douille 22 du réservoir 1 de reprendre sa position initiale d'avant distribution du produit, permettant ainsi notamment de prévenir une réutilisation du dispositif pendant un certain laps de temps. Ces moyens comprennent avantageusement un second crochet de verrouillage 57, visible sur la figure 12, sensiblement identique au premier crochet de verrouillage 31 et qui est disposé symétriquement à ce dernier par rapport à l'axe 51 supportant la culasse mobile 33. Le second crochet de verrouillage 57 est également monté rotatif sur un axe 52 solidaire de la platine 48. Il coopère, par l'intermédiaire de sa tête 57a, avec une seconde goupille 58, solidaire de l'organe de butée 47, et est entraîné dans sa position de libération, permettant à la douille de remonter vers sa position de repos, par un second ergot 59 disposé symétriquement au premier ergot 34 sur la culasse mobile 33.

Avantageusement, la culasse mobile 33 comporte un bras 71, accessible à l'extérieur du boîtier 21 au travers d'une fente 72, pour actionner ladite culasse manuellement et donc déplacer l'organe de verrou 31 dans sa position de libération, indépendamment d'une aspiration dans le conduit d'inhalation.

Comme représenté schématiquement sur la figure 4, le dispositif de l'invention comporte en outre un circuit électronique 12 de commande des bobines 20 de l'électro-aimant 10, ledit circuit électronique 10 comportant une ou plusieurs piles ou batteries 13 d'alimentation électrique et étant relié à l'écran d'affichage 18 pour commander ledit écran d'affichage. En outre, le circuit électronique 12 comporte un capteur de pression 14, adapté à mesurer la dépression créée dans le conduit d'inhalation 5 par l'aspiration d'un patient. Dans l'exemple représenté sur la figure 4, le conduit d'inhalation 5 comporte un orifice 45, qui est relié de façon étanche au capteur de pression 14 par un tube souple 46. Comme représenté sur la figure 9, le capteur de pression peut éventuellement comporter une pastille de silicium 60, soumise sur une face à la pression atmosphérique et sur l'autre face à la pression régnant dans le conduit d'inhalation. La pastille de silicium 60 comporte un point de résistance qui est déséquilibré par la différence entre la pression atmosphérique P₀ et la pression P₁ régnant dans le conduit d'inhalation puis rééquilibré. La pastille de silicium 60 comporte deux électrodes 61 et 62 qui sont reliées au circuit électronique 12 et qui fournissent audit circuit électronique un signal de tension représentatif de la dépression régnant dans le conduit d'inhalation 5. On peut utiliser comme capteur de pression un capteur du type décrit ci-dessus, fabriqué par la société suisse Keller Métrologie, et vendu sous la référence OEM 0,2 bar. Des capteurs de pression similaires peuvent être fournis par la société NOVA (Etats-Unis) (référence PH 0-15), ou par la société ICS (Etats-Unis) (modèle 30).

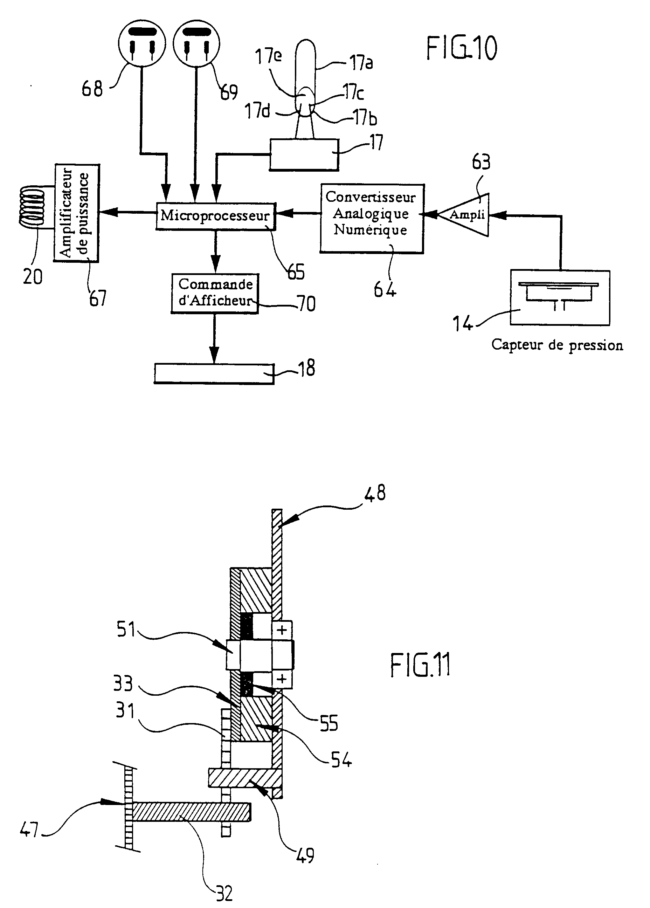

Comme représenté sur la figure 10, le circuit électronique de commande 12 comporte un micro-processeur 65, qui est relié au capteur de pression 14 par l'intermédiaire d'un amplificateur 63 et d'un convertisseur analogique-numérique 64. En outre, le micro-processeur 65 est relié à un amplificateur de puissance 67 qui commande le passage de courant dans les bobines d'auto-induction 20. Le micro-processeur est en outre relié avantageusement à deux contacts 68, 69 qui détectent respectivement l'ouverture complète et la fermeture complète du capot 6. Le micro-processeur 65 est en outre relié à un circuit de commande d'afficheurs 70, lui-même relié à l'écran 18. Enfin, le micro-processeur 65 est relié à un capteur de position 17, qui peut par exemple comporter une colonne de verte creuse 17a dans laquelle on a fait le vide, et dans laquelle circule une goutte de mercure 17e. La colonne 17a comporte une extrémité 17b, qui lorsque l'appareil est dans une position correcte d'utilisation, est une extrémité inférieure, et ladite extrémité inférieure 17b comporte deux contacts 17c, 17d, qui sont mouillés par la goutte de mercure 17e lorsque l'appareil est dans une position correcte d'utilisation. Un tel capteur de position est disponible auprès de la société SAUNDERS (Etats-Unis) sous la référence 188 0001.

Le fonctionnement du dispositif est le suivant. Lorsque le capot 6 est fermé, comme représenté sur les figures 1 et 4, le contact 69 indique au micro-processeur 65 que le capot est fermé et le circuit électronique 12 est désactivé. Lorsque le patient ouvre le capot 6, comme représenté sur la figure 7, le ressort 8 est comprimé, comme il a déjà été expliqué ci-dessus, la douille 22 et le réservoir 1 sont maintenus en place confie la force du ressort 8 par le doigt de verrouillage 9, et le contact 68 indique au micro-processeur 65 que le capot est ouvert, ce qui active le circuit électronique 12, et met en marche l'écran d'affichage 18.

Lorsque le patient aspire dans le conduit d'inhalation 5, la dépression créée dans le conduit d'inhalation est détectée par le capteur de pression 14, qui envoie au microprocesseur 65 un signal d'aspiration, par l'intermédiaire de l'amplificateur 63 et du convertisseur analogique numérique 64. Dans cet exemple, le signal d'aspiration est un signal analogique de tension dont la valeur est proportionnelle à la dépression dans le conduit d'inhalation 5. Le micro-processeur 65 reçoit donc la valeur de la dépression dans le conduit d'inhalation 5, et lorsque ladite pression atteint une valeur prédéterminée, le micro-processeur commande le passage d'une impulsion de courant dans les bobines de l'électro-aimant par l'intermédiaire de l'amplificateur de puissance 67. Le courant qui traverse les bobines d'induction 20 de l'électro-aimant est un courant continu de polarité et d'intensité adaptées à supprimer ou à diminuer le champs magnétique créé par le noyau magnétique 19.

De ce fait, la culasse mobile 33, en équilibre instable face à l'aimant quadripolaire par opposition de pôles identiques, est entraînée en rotation par la force magnétique de répulsion vers sa position d'actionnement, l'ergot 34 poussant l'extrémité du crochet de verrouillage 31, de sorte que sa tête 31a n'interfère plus avec la goupille 32 de l'organe de butée 47, de sorte que la douille 22 peut se déplacer librement vers le bas sous l'effet de ressort de compression 8 en entraînant le réservoir 1. Ainsi, l'organe d'actionnement 3 de la valve doseuse 2 passe de sa position de repos à sa position d'actionnement, comme représenté sur la figure 8. Une dose de substance active est alors pulvérisée dans le conduit d'inhalation 5 par la valve doseuse 2, et cette dose de substance active est inhalée par le patient.

En cas de crise aigüe, nécessitant plusieurs prises à des intervalles de temps très rapprochés, le patient peut aussi actionner manuellement le dispositif en basculant l'organe d'actionnement forcé 71 qui amène ladite culasse mobile 33 dans sa position d'actionnement, ce qui provoque une pulvérisation dans le conduit d'inhalation 5, comme précédemment.

Lorsque l'utilisateur referme le capot 6, le ressort 8 se décomprime et la douille 22 est repoussée vers le haut avec le réservoir 1, sous l'action du ressort de rappel interne (non représenté) de la valve doseuse 2, si le circuit électromagnétique de commande 12 a débloqué le second crochet de verrouillage 57. La tige d'actionnement 3 revient ainsi à sa position de repos, et le crochet de verrouillage 31 revient en position de verrouillage, la tête 31a s'engageant sur la goupille 32.

Avantageusement, le micro-processeur 65 du circuit électronique 12 est programmé pour ne pas déclencher l'actionnement de la valve doseuse 2 si le capteur de position 17 ne détecte pas une position correcte du dispositif. Par exemple, le micro-processeur 65 ne déclenche la pulvérisation que si la goutte 17e de mercure mouille les deux contacts 17d, 17c du capteur de position. Ainsi, on évite de pulvériser dans le conduit d'inhalation 5 une dose de gaz propulseur sans produit actif, ou on évite de désamorcer la valve doseuse 2. L'utilisation d'un capteur de position n'est pas limitée à un dispositif ayant une valve doseuse fonctionnant en position inversée, comme dans le cas présent, mais s'applique à tous dispositifs ayant une position préférentielle de fonctionnement.

En outre, il est nécessaire que l'orientation du dispositif soit correcte lorsque la tige d'actionnement passe de sa position d'actionnement à sa position de repos, pour ne pas désamorcer la valve. Pour cette raison, si le capteur d'inclinaison 17 détecte une mauvaise orientation au moment où le capot est refermé (lorsque les contacts 68 et 69 sont tous les deux désactivés) après un actionnement du dispositif, l'écran 18 affiche un message qui avertit le patient que la dose suivante sera incomplète. Le message affiché peut aussi demander à l'utilisateur d'actionner manuellement une fois le dispositif au moyen du levier 71, pour réamorcer la valve doseuse. Si le circuit 12 compte les doses émises, cet actionnement manuel n'est de préférence pas compté comme une dose émise.

Avantageusement, le micro-processeur 65 ne déclenche la pulvérisation que si le dispositif a été secoué au cours d'une durée prédéterminée T₂ précédent l'inhalation du patient. L'agitation du dispositif est avantageusement détectée au moyen du capteur de position 17, puisqu'une agitation du dispositif provoque des mouvements de la goutte 17e de mercure, ce qui se traduit par une série de rupture de contact électrique entre les contacts 17c et 17d.

Avantageusement, le micro-processeur 65 est programmé pour ne pas déclencher l'actionnement du dispositif tant qu'il ne s'est pas écoulé une durée prédéterminée T₁ depuis le dernier actionnement, pour éviter les surdosages. Eventuellement, le micro-processeur 65 peut être programmé pour compter le nombre de doses émises, et pour ne pas déclencher l'actionnement si le dispositif a déjà émis un nombre prédéterminé N₁ de doses au cours d'une période prédéterminée T₃.

Avantageusement, le micro-processeur 65 a en mémoire le nombre maximal de doses de substance active contenue dans le réservoir 1, le micro-processeur 65 compte le nombre de doses émises par le dispositif, et ledit micro-processeur émet un signal perceptible par le patient, par exemple un message affiché par l'écran 18, lorsque le nombre de dose émises atteint un seuil prédéterminé voisin du nombre maximal de doses contenues dans le réservoir 1.

Avantageusement, lorsqu'on retourne le dispositif de façon que le conduit d'inhalation 5 se trouve au-dessus du réservoir 1, ce qui est détecté par le capteur de position 17, le micro-processeur 65 est progammé pour fonctionner en mode d'étalonnage, auquel cas il n'actionne pas le dispositif lorsque le patient aspire dans le conduit d'inhalation 5, mais il détermine la dépression maximale ΔPₘ créée dans le conduit d'inhalation au cours de l'apiration, puis ledit micro-processeur 65 calcule et mémorise une dépression de déclenchement ΔP₀ en fonction de la dépression maximale mesurée (par exemple, un certain pourcentage de ΔPₘ), de sorte que le dispositif est ultérieurement actionné, lorsqu'il est en position d'utilisation correcte, si une dépression supérieure ou égale à ladite dépression de déclenchement ΔP₀ est mesurée dans le conduit d'inhalation par le capteur de pression 14. Avantageusement, dans ce cas, le micro-processeur 65 est programmé pour calculer le débit d'air inhalé par le patient en fonction de la dépression mesurée dans le conduit d'inhalation 5, et ledit micro-processeur affiche le débit d'air inhalé sur l'écran 18, de sorte que l'appareil peut aussi servir de spiromètre.

Lorsque le réservoir 1 est vide, il est possible de remplacer ledit réservoir 1 avec sa valve doseuse 2, par un réservoir 1 neuf. Pour cela, lorsque le capot 6 est fermé, comme représenté sur la figure 1, on appuie légèrement sur la partie supérieure du capot 6 en sollicitant en même temps ladite partie supérieure 6b en direction de l'embout 37. De cette façon les pivots 26 du capot 6 s'engagent dans les canaux de démontage 43, puis on dégage les pivots 26 par les puits de sortie 44 (voir figure 6). Le capot 6 est alors séparé du boîtier 21, et le boîtier 21 comporte une ouverture 30 qui est dégagée lorsque le capot 6 est démonté, l'ouverture 30 étant de taille suffisante pour permettre d'accéder à l'intérieur du boîtier 21 et de sortir la douille 22, le réservoir 1 et sa valve doseuse 2, en déboîtant la tige d'actionnement 3 hors de l'alésage 39. Avantageusement, comme représenté sur la figure 4b, l'extrémité libre de la paroi de guidage 24 comporte une lèvre intérieure 24a, qui maintient le piston 7 dans ladite paroi de guidage 24 lorsque le piston 7 n'est plus en contact avec la surface d'appui 91 du capot 6.

Après avoir sorti le réservoir 1 et sa valve doseuse 2, on déboîte la douille 22 et on la remboîte sur le fond d'un nouveau réservoir 1, puis on remet l'ensemble en place dans le boîtier et on remonte le capot 6 sur le boîtier 21.

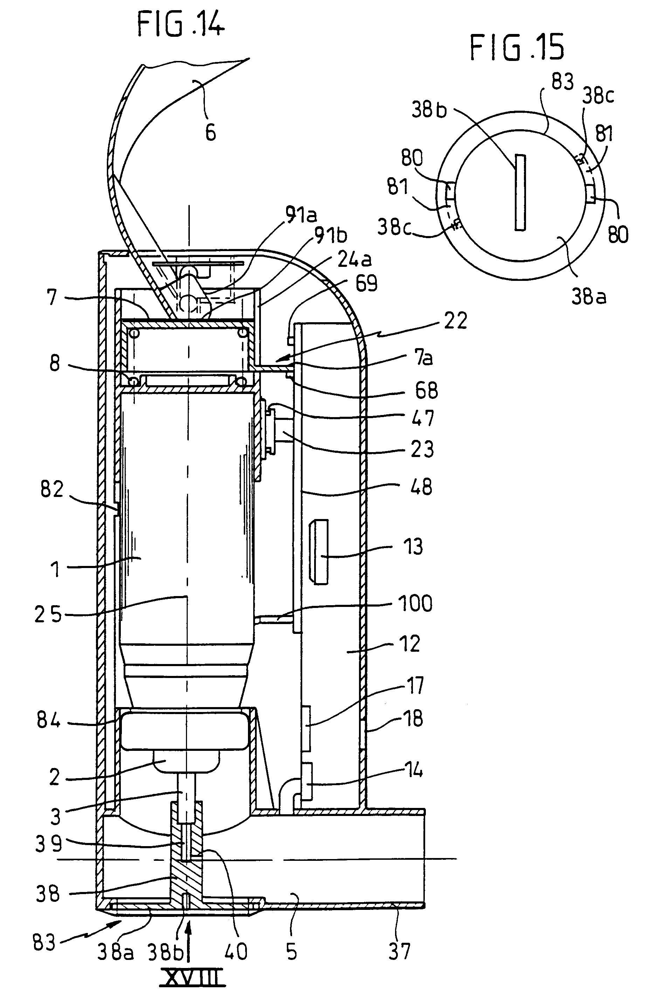

Les figures 14 et 15 représentent une variante du dispositif des figures précédentse, qui ne sera pas décrite en détail ici. Sur les figures 14 et 15, les parties identiques ou similaires à celles du dispositif de sfigures précédentes sont désignées par les mêmes références.

Le dispositif des figures 14 et 15 se distingue du dispositif des figures précédentes en ce que le boîtier 21 comporte en partie inférieure une ouverture 83, de taille suffisante pour permettre le passage du réservoir 1 avec sa valve 2. Le réservoir 1 et la valve 2 peuvent coulisser librement le long de l'axe 25 lors de la sortie du réservoir 1, sans interférer avec le boîtier 21. Le plot 38 déjà décrit est solidaire d'un disque 38a monté amovible dans l'ouverture 83. Dans l'exemple réprésenté le disque 38a comporte deux ergots 38c qui saillent radialement vers l'extérieur, et qui peuvent s'engager dans deux rainures 81 ouvertes radialement vers l'intérieur de l'ouverture 83. Les rainures 81 communiquent en outre chacune avec une ouverture axiale 80, dirigée vers le bas, pour recevoir les ergots 38c, qui sont ensuite engagés dans les rainures 80 par rotation. La rotation du disque 38a peut se faire au moyen d'une pièce de monnaie ou d'un tourne-vis qu'on engage dans une fente 38b du disque 38a. Lors de la sortie du réservoir 1, la douille 22 est normalement maintenue en place par le mécanisme de verrouillage mais on peut en outre prévoir éventuellement une butée 82 solidaire du boîtier 21, pour empêcher la descente de la douille 22 avec le réservoir 1.

Un avantage de la variante des figures 14 et 15, est qu'elle permet le remplacement du plot 38 à chaque changement de réservoir 1, ce qui garantit une bonne hygiène de l'appareil, et notamement une bonne propreté de l'orifice latéral 40 de pulvérisation.

Sur la figure 14, l'orifice d'entrée d'air a été supprimé l'air pénétrant dans le conduit d'inhalation 5 par l'intérieur du boitier 21, en passant autour de la valve 2 et du réservoir 1, qui coulissent sans étanchéité dans un puits de guidage 84, ce qui simplifie le moulage du boîtier et évite l'emploi d'un filtre d'air.

De plus, la paroi de guidage 24 de la figure 14 comporte une fente verticale 24a dans laquelle coulisse un appendice 7a du piston 7, qui interfère avec deux interrupteurs 68, 69 qui constituent les contacts d'ouverture et de fermeture du capot 6. Cette disposition avantageuse peut être utilisée dans la forme de réalisation des figures 1 à 13.

Enfin, le circuit électronique 12 de la figure 14 comporte un capteur 100 pour détecter la présence du réservoir 1 ou de la valve doseuse 2. Le capteur 100 peut être un contact électrique ou un capteur à effet de champ (capteur de proximité). Le capteur 100 peut permettre la remise à zéro du compteur de doses lorsqu'on remplace le réservoir 1 et sa valve doseuse 2. Le capteur 100 permet aussi au circuit 12 de compter le nombre de réservoirs 1 déjà utilisés, pour garantir que l'appareil n'a jamais été utilisé lorsqu'il est acheté vide (sans le réservoir 1 et sa valve 2). Le comptage du nombre de réservoirs 1 déjà utilisés peut ausi permettre de bloquer le fonctionnement de l'appareil après un certain nombre de réservoir 1 utilisés. Le capteur 100 peut aussi bloquer le fonctionnement de l'appareil s'il ne détecte pas la présence du réservoir 1. Le capteur 100 peut être utilisé dans la forme de réalisation des figures 1 à 13.

## Revendications

1. Dispositif portatif de pulvérisation à actionnement déclenché par l'inhalation, comportant :
- une réserve (1) d'une substance à pulvériser,
- un dispositif de distribution (2) ayant un organe d'actionnement (3) mobile entre une position de repos et une position d'actionnement, ledit dispositif de distribution (2) émettant une dose mesurée de ladite substance, lorsque l'organe d'actionnement est déplacé de sa position de repos à sa position d'actionnement, ledit organe d'actionnement (3) étant rappelé élastiquement vers sa position de repos, le dispositif de distribution (2) ayant en outre une sortie (4) pour émettre ladite substance,
- un conduit d'inhalation (5) dans lequel un patient peut aspirer de l'air, et qui communique avec ladite sortie (4) de ladite substance,
- des moyens de sollicitation (6, 7, 8) de l'organe d'actionnement pour solliciter ledit organe d'actionnement (3) vers sa position d'actionnement,
- un organe de verrou (31) mobile entre une position de verrouillage, où il bloque l'action desdits moyens de sollicitation, et une position de libération , où il ne bloque plus l'action desdits moyens de sollicitation,
- des moyens de déblocage (10, 11, 12, 13, 14) pour déplacer ledit organe de verrou vers sa position de libération, lors d'une aspiration dans le conduit d'inhalation (5), lesdits moyens de déblocage comportant :
- un actionneur électrique (10) pour déplacer ledit organe de verrou (9),
- un circuit électronique de commande (12) connecté audit actionneur électrique pour le commander,
- une source d'énergie (13) pour alimenter ledit circuit électronique de commande, et
- un capteur d'aspiration (14) pour envoyer audit circuit électronique de commande un signal d'aspiration, lorsqu'il détecte une aspiration dans le conduit d'inhalation (5), le circuit électronique de commande (12) commandant alors l'actionneur électrique (10) pour déplacer l'organe de verrou (9) dans sa position de libération,
le dispositif étant caractérisé en ce qu'il comporte en outre un capteur de position (17) pour détecter une orientation correcte dudit dispositif, dans lequel ledit capteur de position (17) est connecté audit circuit électronique de commande (12), et ledit circuit électronique de commande (12) est adapté à ne déclencher l'actionnement du dispositif que si ledit capteur de position (17) détecte ladite orientation correcte du dispositif.

2. Dispositif selon la revendication 1, dans lequel ledit capteur d'aspiration (14) mesure une dépression dans ledit conduit d'inhalation par rapport à la pression atmosphérique et envoie au circuit électronique de commande (12) un signal représentatif de la dépression.

3. Dispositif selon une des revendications 1 ou 2, dans lequel ledit capteur de position (17) comporte des moyens (17a-e) pour détecter une position particulière du dispositif et faire fonctionner ledit circuit électronique de commande (12) en mode d'étalonnage, auquel cas le dispositif n'est pas actionné lorsque le patient aspire dans le conduit d'inhalation (5), mais ledit circuit électronique de commande détermine alors la dépression maximale (ΔPₘ) créée dans le conduit d'inhalation (5) au cours de l'aspiration, et calcule et mémorise une dépression de déclenchement (ΔPo) en fonction de ladite dépression maximale (ΔPm), le dispositif étant ultérieurement actionné lorsqu'une dépression supérieure ou égale à ladite dépression de déclenchement (ΔPo) est détectée dans le conduit d'inhalation (5) et lorsque le circuit électronique de commande (12) ne fonctionne plus en mode d'étalonnage.

4. Dispositif selon la revendication 2 ou 3, comportant en outre des moyens d'affichage (18) connectés au circuit électronique de commande (12) pour afficher une valeur de débit d'air inhalé par le patient en fonction de la dépression mesurée dans le conduit d'inhalation (5).

5. Dispositif selon l'une quelconque des revendications précédentes comportant un capteur d'agitation (17) pour détecter une agitation de ladite réserve (1), dans lequel ledit capteur d'agitation est connecté audit circuit électronique de commande (12), et ledit circuit électronique de commande (12) est adapté à ne pas déclencher l'actionnement du dispositif si ladite réserve (1) n'a pas été agitée au cours d'une deuxième durée prédéterminé (T2) précédant l'inhalation du patient.

6. Dispositif selon l'une quelconque des revendications précédentes, dans lequel le circuit électronique de commande (12) est connecté à des moyens avertisseurs (18) et ledit circuit électronique de commande (12) est adapté à commander les moyens avertisseurs (18) pour prévenir le patient que le dispositif n'est pas dans une position correcte d'utilisation, si le capteur de position (17) ne détecte pas ladite position correcte.

7. Dispositif selon l'une quelconque des revendications précédentes, dans lequel la substance à pulvériser est liquide ou semi-liquide le dispositif de distribution (2) est une pompe ou une valve aérosol, le circuit électronique de commande (12) est connecté à des moyens (14, 68, 69) pour détecter le déplacement de l'organe d'actionnement (3) de sa position d'actionnement à sa position de repos, le circuit électronique de commande (12) est connecté à des moyens avertisseurs (18) et ledit circuit électronique de commande (12) est adapté à commander les moyens avertisseurs (18) pour prévenir le patient que la dose suivante de ladite substance qui sera délivrée par le dispositif, sera incomplète, dans le cas où ledit déplacement de l'organe d'actionnement (3) est détecté lorsque le dispositif n'est pas dans son orientation correcte.

8. Dispositif selon la revendication 7, comportant des moyens (71) pour actionner manuellement la pompe ou la valve (2), et dans lequel lesdits moyens avertisseurs (18) indiquent au patient d'actionner manuellement une fois la pompe ou la valve (2).

9. Dispositif selon une des revendications 6 à 8, comportant des moyens pour compter les doses de substance émises, et dans lequel l'émission de ladite dose suivante n'est pas comptée.

10. Dispositif selon l'une quelconque des revendications précédentes, dans lequel ledit capteur de position (17) comporte une colonne creuse (17a) qui est orientée verticalement lorsque le dispositif est dans sa position correcte, ladite colonne creuse (17a) ayant une extrémité inférieure (17b) dotée de deux contacts électriques (17c, 17d) et ladite colonne creuse (17a) contenant une goutte de mercure (17e) qui mouille les deux contacts électriques (17c, 17d) lorsque le dispositif est dans sa position correcte.

11. Dispositif selon la revendication 10, dans lequel ledit capteur de position (17) sert aussi de capteur d'agitation, et ledit circuit électronique de commande (12) est adapté à ne pas déclencher l'actionnement du dispositif si ladite réserve (1) n'a pas été agitée au cours d'une durée prédéterminée (T₂) précédant l'inhalation du patient.

12. Dispositif selon l'une quelconcque des revendications précédentes, dans lequel ledit actionneur électrique comporte un électro-aimant (10), l'organe de verrou (31) étant en liaison mécanique avec une culasse mobile (33) sensible aux champs magnétiques, ladite culasse mobile (33) étant disposée face audit électro-aimant (10), ledit électro-aimant (10) comporte un noyau (19) à aimantation permanente, qui maintient ladite culasse (33) dans une position d'attente, ledit électro-aimant (10) comporte en outre une bobine (20), et le circuit électronique de commande (12) est adapté à alimenter ladite bobine (20) par un courant de polarisation et d'intensité adaptées à faire passer ladite culasse mobile (33) dans une position d'actionnement, ladite culasse mobile (33) entraînant ainsi l'organe de verrou (31) de sa position de verrouillage à sa position de libération.

13. Dispositif selon la revendication 12, dans lequel la culasse mobile (33) comporte au moins un ergot (34) coopérant avec ledit organe de verrou (31), ladite culasse mobile (33) étant montée rotative sur un axe (51) et ledit organe de verrou (31) étant monté rotatif sur un axe (49), lesdits axes (49) et (51) étant solidaires d'une platine (48), l'ergot (34) entraînant l'organe de verrou (31) en rotation, de sa position de verrouillage, où il coopère avec une goupille (32) solidaire de la réserve de produit (1), à sa position de libération.

14. Dispositif selon la revendication 13, dans lequel l'électro-aimant (10) est quadripolaire et la culasse mobile (33) est maintenue en équilibre instable par opposition de pôles identiques dans sa position de verrouillage, un signal du circuit électronique de commande (12) diminuant la force d'attraction de l'électro-aimant (10), de sorte que la répulsion magnétique créée par l'opposition de pôles entraîne ladite culasse (33) en rotation vers sa position d'actionnement.

15. Dispositif selon l'une des revendications 12 à 14, dans lequel est prévu un second organe de verrou (57), monté rotatif sur un axe (52) solidaire de la platine (48), sensiblement identique à l'organe de verrou (31) et disposé symétriquement audit organe de verrou (31) par rapport a l'axe (51) supportant la culasse mobile (33), ledit second organe de verrou (57) coopérant avec une seconde goupille (58) solidaire de la réserve de produit (1) et étant entraîné dans sa position de libération par un second ergot (59) disposé symétriquement au premier ergot (34) sur la culasse mobile (33), ledit second organe de verrou (57) empêchant le dispositif de distribution (2) de retourner dans sa position de repos.

16. Dispositif selon l'une des revendications 12 à 15, dans lequel la culasse mobile (33) comporte un bras (71) s'étendant en dehors du boîtier (21) pour actionner manuellement ladite culasse mobile (33), et donc déplacer l'organe de verrou (31) dans sa position de libération, indépendamment d'une aspiration dans le conduit d'inhalation.

17. Dispositif selon l'une quelconque des revendications précédentes, dans lequel le dispositif de distribution (2) est solidaire de la réserve (1) de substance à pulvériser, la position d'actionnement de l'organe d'actionnement (3) est telle que ledit organe d'actionnement (3) est rapproché de ladite réserve (1) pour passer de la position de repos à la position d'actionnement, le dispositif portatif de pulvérisation comporte un boîtier (21) solidaire du conduit d'inhalation (5) et de l'organe d'actionnement (3), le dispositif portatif de pulvérisation comporte en outre un capot (6) articulé sur ledit boîtier (21) et mobile entre une position fermée, où il ferme le conduit d'inhalation (5), et une position ouverte où il ouvre le conduit d'inhalation (5), le dispositif portatif de pulvérisation comporte en outre un ressort de compression (8) adapté à solliciter la réserve (1) vers l'organe d'actionnement (3) pour déplacer ledit organe d'actionnement (3) dans sa position d'actionnement, ledit ressort de compression (8) ayant une première extrémité qui agit sur la réserve (1) et une deuxième extrémité qui agit sur un organe d'appui (7), le capot (6) comporte une partie formant levier (6a) qui appuie sur l'organe d'appui (7) lorsque le capot (6) est déplacé dans sa position ouverte, en comprimant le ressort de compression (8) et ledit organe de verrou (31) bloque la réserve (1) lorsqu'il est dans sa position de verrouillage, lit première extrémité dit ressort de compression (8) agit sur un organe intermédiaire (22) fixé à ladite réserve (1) et ledit organe intermédiaire (22) comporte une goupille (32) pour recevoir ledit organe de verrou (31) lorsqu'il est dans sa position de verrouillage, ledit organe intermédiaire (22) collaborant alors avec l'organe de verrou (31) pour bloquer la réserve (1).

18. Dispositif selon la revendication 17, dans lequel l'organe d'actionnement (3) est déplaçable relativement ait dispositif de distribution (2) parallèlement à un axe (25), le capot (6) est monté rotatif autour de deux pivots (26) et les pivots (26) sont montés chacun dans un chemin de guidage (27) allongé parallèle à l'axe (25), les pivots (26) étant ainsi déplaçables axialement dans leurs chemins de guidage (27) contre la force du ressort de compression, et le capot (6) comporte en outre deux saillies latérales (28) dirigées vers l'extérieur, qui collaborent chacune avec une surface saillante (29) du boîtier dirigée vers l'intérieur, pour obliger les pivots (26) à se déplacer dans leurs chemins de guidage (27) en comprimant le ressort de compression, (8) lorsque le capot (6) passe de sa position fermée à sa position ouverte.

19. Dispositif selon la revendication 18, dans lequel les deux chemins de guidage (27) comportent deux extrémités axiales et présentent chacun une ouverture (43, 44) disposée au même niveau sur les deux chemins de guidage, à une position intermédiaire entre les deux extrémités axiales de chemins de guidage, pour permettre la sortie des ergots (26) hors de chemins de guidage lorsqu'on appuie sur le capot (6) parallèlement à l'axe (25) et qu'en même temps on sollicite le capot (6) perpendiculairement à l'axe (25) dans la direction desdites ouvertures, le capot (6) étant ainsi démontable du boîtier (21), le boîtier (21) comportant une ouverture de chargement (30) de taille permettant le passage de la réserve de substance (1) avec son dispositif de distribution (2) lorsque le capot (6) est démonté du boîtier, l'organe d'actionnement du dispositif de distribution (2) étant fixé de façon amovible audit boîtier (21), et le boîtier comporte des moyens (71) pour déplacer l'organe de verrou (31) dans sa position de libération, indépendamment d'une aspiration dans le conduit d'inhalation (5).

20. Dispositif selon l'une quelconque des revendications 17 à 19, dans lequel ladite partie formant levier (6a) du capot (6) présente une surface d'appui (91) en contact avec l'organe d'appui (7) et ladite surface d'appui (91) comporte deux méplats (91a, 91b) s'appuyant sur l'organe d'appui (7) respectivement dans la position fermée et dans la position ouverte du capot.

21. Dispositif selon l'une quelconque des revendications 17 à 20, dans lequel le boîtier (21) comporte une ouverture (83) de taille adaptée à permettre le passage de la réserve (1) avec son dispositif de distribution (2) pour la rentrer dans le boîtier (21) ou la sortir du boîtier et ledit boîtier (21) comporte en outre un organe de support (38) qui est solidaire de l'organe d'actionnement (3) et qui est monté amovible dans ladite ouverture (83).

22. Dispositif selon l'une quelconque des revendications précédentes, comportant en outre un capteur (100) pour détecter la présence de la réserve (1) et/ou du dispositif de distribution (2), ledit capteur (100) étant connecté au circuit électronique de commande (12) pour empêcher le fonctionnement du dispositif en cas d'absence de ladite réserve (1).

23. Dispositif selon l'une quelconque des revendications précédentes, comportant en outre un capteur (100) pour détecter la présence de la réserve (1) et/ou du dispositif de distribution (2), ledit capteur (100) étant connecté au circuit électronique de commande (12), ledit circuit électronique de commande est adapté à compter le nombre de réserves (1) qui ont été mises en place dans ledit dispositif en fonction des indications dudit capteur (100) de détection de présence.

24. Dispositif selon la revendication 23, dans lequel ledit circuit électronique de commande est adapté à empêcher tout actionnement du dispositif si ledit nombre de réserves (1) qui ont été mises en place dans le dispositif dépasse un nombre prédéterminée.

## Claims

1. A hand-held spray device actuated by inhaling, the device comprising:
• a tank (1) of substance to be sprayed;
• a dispenser device (2) having an actuator member (3) that is movable between a rest position and an actuated position, said dispenser device (2) issuing a measured amount of said substance when the actuator member is displaced from its rest position to its actuated position, said actuator member (3) being urged resiliently towards its rest position, the dispenser device (2) also having an outlet (4) for issuing said substance;
• an inhale duct (5) through which a patient can suck in air, and which communicates with said outlet (4) for said substance;
• actuator member urging means (6, 7, 8) for urging said actuator member (3) towards its actuated position;
• a latch member (31) movable between a latching position in which it locks said actuator member urging means, and a released position in which it no longer locks said urging means; and
• unlocking means (10, 11, 12, 13, 14) for displacing said latch member towards its released position while suction is being applied to said inhale duct (5);
said unlocking means comprising:
• an electrical actuator (10) for displacing said latch member (9);
• an electronic control circuit (12) connected to said electrical actuator for the purpose of controlling it;
• a source of energy (13) for powering said electronic control circuit; and
• a suction sensor (14) for applying a suction signal to said electronic control circuit on detecting suction in the inhale duct (5), the electronic control circuit (12) then causing the electrical actuator (10) to displace the latch member (9) into its released position;
the device being characterized in that it further includes a position sensor (17) for detecting proper orientation of said device, in which said position sensor (17) is connected to said electronic control circuit (12), and said electronic control circuit (12) is adapted to trigger actuation of the device only in the event of said position sensor (17) detecting that the device is in said proper orientation.

2. A device according to claim 1, in which said suction sensor (14) measures suction in said inhale duct relative to atmospheric pressure, and delivers a signal representative of the suction to the electronic control circuit (12).

3. A device according to claim 1 or 2, in which said position sensor (17) includes means (17a-e) for detecting a particular position of the device and for causing said electronic control circuit (12) to operate in a calibration mode, in which case the device is not actuated when the patient sucks from the inhale duct (5), but instead said electronic control circuit then determines the maximum amount of suction (ΔPₘ) set up in the inhale duct (5) during suction, and it calculates and stores a trigger level of suction (ΔP₀) as a function of said maximum suction (ΔPₘ), the device subsequently being actuated only when suction greater than or equal to said trigger level of suction (ΔP₀) is detected in the inhale duct (5), and providing said electronic control circuit (12) is not operating in its calibration mode.

4. A device according to claim 2 or 3, further including display means (18) connected to the electronic control circuit (12) to display a value for the air flow inhaled by the patient as a function of the suction measured in the inhale duct (5).

5. A device according to any preceding claim, including a shaking sensor (17) for detecting shaking of said tank (1), in which said shaking sensor is connected to said electronic control circuit (12) and said electronic control circuit (12) is adapted to inhibit actuation of the device in the event of said tank (1) not having been shaken during a second predetermined duration (T₂) preceding inhalation by the patient.

6. A device according to any preceding claim, in which the electronic control circuit (12) is connected to warning means (18) and said electronic control circuit (12) is adapted to switch on the warning means (18) to warn the patient that the device is not in a proper position for use, in the event that the position detector (17) is not detecting the said proper position.

7. A device according to any preceding claim, in which the substance to be sprayed is a liquid or a semi-liquid, the dispenser device (2) is an aerosol valve or a pump, the electronic control circuit (12) is connected to means (14, 68, 69) for detecting displacement of the actuator member (3) from its actuated position to its rest position, the electronic control circuit (12) is connected to warning means (18), and said electronic control circuit (12) is adapted to switch on the warning means (18), in the event that said displacement of the actuator member (3) is detected while the device is not in its proper position, to warn the patient that the next dose of said substance that the device dispenses will be incomplete.

8. A device according to claim 7, including means (71) for actuating the valve (2) or the pump manually, and in which said warning means (18) tell the patient to actuate the valve (2) or the pump manually for one occasion.

9. A device according to any one of claims 6 to 8, including means for counting the number of doses of substance that are issued, and in which the issuing of said next dose is not counted.

10. A device according to any preceding claim, in which said position sensor (17) includes a hollow column (17a) that extends vertically when the device is in its proper position, said hollow column (17a) having a bottom end (17b) provided with two electrical contacts (17c, 17d) and said hollow column (17a) containing a drop of mercury (17e) which wets the two electrical contacts (17c, 17d) when the device is in its proper position.

11. A device according to claim 10, in which said position sensor (17) also serves as a shaking sensor, and said electronic control circuit (12) is adapted to inhibit actuation of the device if said tank (1) has not been shaken during a predetermined length of time (T₂) preceding inhalation by the patient.

12. A device according to any preceding claim, in which said electrical actuator includes an electromagnet (10), the latch member (31) being in mechanical connection with an armature (33) that is sensitive to magnetic fields, said armature (33) being disposed facing said electromagnet (10), said electromagnet (10) including a permanently magnetized core (19) for maintaining said armature in a waiting position, said electromagnet (10) further including a coil (20), and said electronic control circuit (12) being adapted to power said coil with a current whose direction and magnitude are adapted to cause said armature (33) to move into an actuated position, said armature (33) thus driving the latch member (31) from its latching position into its released position.

13. A device according to claim 12, in which the armature (33) includes at least one stud (34) co-operating with said latch member (31), said armature (33) being mounted to rotate on an axis (51) and said latch member (31) being mounted to rotate on an axis (49), said axes (49 and 51) being secured to a plate (48), the stud (34) driving the latch member (31) in rotation from its latching position where it co-operates with a pin (32) secured to the tank of substance (1) to its released position.

14. A device according to claim 13, in which the electromagnet (10) is a four-pole magnet and the armature (33) is held in unstable equilibrium by opposing forces between identical poles while it is in its latching position, a signal from the electronic control circuit (12) reducing the attraction force of the electromagnet (10) in such a manner that the magnetic repulsion created by opposition between poles causes said armature (33) to rotate towards its actuated position.

15. A device according to any one of claims 12 to 14, in which a second latching member (57) is provided that is rotatably mounted on an axis (52) secured to the plate (48), which member is substantially identical to the latch member (31) and is disposed symmetrically relative to said latch member (31) about the axis (51) supporting the armature (33), said second latch member (57) co-operating with a second pin (58) secured to the tank of substance (1) and being driven into its released position by a second stud (59) disposed symmetrically to the first stud (34) on the armature (33), said second latch member (57) preventing the dispenser device (2) from returning to its rest position.

16. A device according to any one of claims 12 to 15, in which the armature (33) includes an arm (71) extending outside the housing (21) to enable said armature (33) to be actuated manually, thereby enabling the latch member (31) to be moved into its released position independently of any suction in the inhale duct.

17. A device according to any preceding claim, in which the dispenser device (2) is secured to the tank (1) of substance to be sprayed, the actuated position of the actuator member (3) being such that said actuator member (3) is moved towards said tank (1) on passing from its rest position to its actuated position, the hand-held spray device including a housing (21) secured to the inhale duct (5) and to the actuator member (3), the hand-held spray device further including a cover (6) hinged on said housing (21) and movable between a closed position in which it closes the inhale duct (5), and an open position in which it opens the inhale duct (5), the hand-held spray device further including a compression spring (8) adapted to urge the tank (1) towards the actuator member (3) to displace said actuator member (3) into its actuated position, said compression spring (8) having a first end which acts on the tank (1) and a second end which acts on a thrust member (7), the cover (6) including a lever-forming portion (6a) which bears against the thrust member (7) when the cover (6) is displaced into its open position, compressing the compression spring (8) and said latch member (31) locks the tank (1) when it is in its latching position, the first end of the compression spring (8) acts on an intermediate member (22) fixed to said tank (1) and said intermediate member (22) includes a pin (32) for receiving said latch member (31) when it is in its latching position, said intermediate member (22) then co-operating with the latch member (31) to lock the tank (1).

18. A device according to claim 17, in which the actuator member (3) is displaceable relative to the dispenser member (2) parallel to an axis (25), the cover (6) is mounted to rotate about two pivots (26), and the pivots (26) are mounted in respective elongate guide paths (27) parallel to the axis (25), the pivots (26) thus being axially displaceable along their guide paths (27) against the force of the compression spring, and the cover (6) further including two lateral projections (28) that are outwardly directed, and that co-operate with respective inwardly-directed projecting surfaces (29) of the housing to constrain the pivots (26) to move along their guide paths (27) while compressing the compression spring (8) when the cover (6) passes from its closed position to its open position.

19. A device according to claim 18, in which the two guide paths (27) include two axial ends and each has an opening (43, 44) disposed at the same level on both guide paths in a position that is intermediate between the two axial ends of each of the guide paths, to enable the studs (26) to escape from the guide paths when pressure is applied to the cover (6) parallel to the axis (25), and simultaneously the cover (6) is urged parallel to the axis (25) towards said openings, the cover (6) thus being removable from the housing (21), the housing (21) including a loading opening (30) of a size that enables the tank of substance (1) together with its dispenser device (2) to pass therethrough once the cover (6) has been removed from the housing, the actuator member for the dispenser device (2) being secured in removable manner to said housing (21), and the housing includes means (71) for displacing the latch member (31) into its released position independently of suction in the inhale duct (5).

20. A device according to any one of claims 17 to 19, in which said lever-forming portion (6a) of the cover (6) has a thrust surface (91) in contact with the thrust member (7) and said thrust surface (91) includes two flats (91a, 91b) bearing against the thrust member (7) respectively in the closed position and in the open position of the cover.

21. A device according to any one of claims 17 to 20, in which the housing (21) includes an opening (83) of appropriate size to allow the tank (1) together with its dispenser device (2) to pass therethrough in order to be inserted into the housing (21) or to be removed from the housing, and said housing (21) further includes a support member (38) which is secured to the actuator member (3) and which is removably mounted in said opening (83).

22. A device according to any preceding claim, further including a sensor (100) for detecting the presence of the tank (1) and/or the dispenser device (2), said sensor (100) being connected to the electronic control circuit (12) to prevent operation of the device in the event of said tank (1) being absent.

23. A device according to any preceding claim, further including a sensor (100) for detecting the presence of the tank (1) and/or of the dispenser device (2), said sensor (100) being connected to the electronic control circuit (12), said electronic control circuit being adapted to count the number of tanks (1) that have been installed in said device as a function of information from said sensor (100) for detecting the presence thereof.

24. A device according to claim 23, in which said electronic control circuit is adapted to prevent the device being actuated if the number of tanks (1) that have been installed in the device exceeds a predetermined number.

## Patentansprüche

1. Tragbare Zerstäubungssvorrichtung, deren Betätigung durch Inhalation ausgelöst wird und die folgende Bestandteile umfaßt:
- einen Vorratsbehälter (1) für eine zu zerstäubende Substanz,
- eine Abgabevorrichtung (2), die ein zwischen einer Ruhelage und einer Betätigungslage bewegliches Betätigungsorgan (3) umfaßt, wobei die Abgabevorrichtung (2) eine abgemessene Dosis der besagten Substanz emmitiert, wenn das Betätigungsorgan aus seiner Ruhelage in seine Betätigungslage verschoben wird, wobei das Betätigungsorgan (3) in elastischer Weise in seine Ruhelage zurückgeholt wird und wobei die Abgabevorrichtung (2) im übrigen einen Ausgang (4) aufweist, um besagte Substanz zu emmitieren,
- eine Inhalationsleitung (5), durch die ein Patient Luft einatmen kann und die mit dem Ausgang (4) für die Substanz in Verbindung steht,
- Belastungsmittel (6,7,8) für das Betätigungsorgan, um das Betätigungsorgan (3) zu seiner Betätigungsstellung hin vorzuspannen,
- ein Verriegelungsorgan (31), das zwischen einer Verriegelungsstellung, in der es die Wirkung der Belastungsmittel blockiert, und einer Freigabeposition beweglich ist, in welcher es die Wirkung der Belastungsmittel nicht mehr blockiert,
- Deblockiermittel (10,11,12,13,14) zum Verschieben des Verriegelungsorgans in seine Freigabeposition bei einem Ansaugen durch die Inhalationsleitung (5), wobei die Deblockiermittel folgende Bestandteile umfassen:
- eine elektrische Betätigungseinrichtung (10) zum Verschieben dieses Verriegelungsorgans (9)
- eine elektronische Steuerschaltung (12), die mit der elektrischen Betätigungseinrichtung verbunden ist, um diese zu steuern,
- eine Energiequelle (13), um die elektronische Steuerschaltung mit Energie zu versorgen, und
- einen Ansaug-Sensor (14), der dazu dient, der elektronischen Steuerschaltung ein Ansaugsignal zuzuführen, wenn er in der Inhalationsleitung (5) einen Ansaugvorgang detektiert, worauf die elektronische Steuerschaltung (12) die elektrische Betätigungseinrichtung (10) ansteuert, um das Verriegelungsorgan (9) in seine Freigabestellung zu verschieben,
dadurch gekennzeichnet, daß die Vorrichtung weiterhin einen Positionssensor (17) umfaßt, der dazu dient, die richtige Orientierung der Vorrichtung zu detektieren, wobei der Positionssensor (17) mit der elektronischen Steuerschaltung (12) verbunden und die elektronische Steuerschaltung (12) so ausgebildet ist, daß sie eine Betätigung der Vorrichtung nur dann auslöst, wenn der Positionsdetektor (17) die korrekte Ausrichtung der Vorrichtung detektiert.

2. Vorrichtung nach Anspruch 1, bei der der Ansaug-Sensor (14) einen Unterdruck in der Inhalationsleitung bezüglich des Atmosphärendrucks mißt und der elektronischen Steuerschaltung (12) ein Signal übermittelt, das für den Unterdruck repräsentativ ist.

3. Vorrichtung nach einem der Ansprüche 1 oder 2, bei der der Positionssensor (17) Mittel (17a-e) umfaßt, um eine spezielle Position der Vorrichtung zu detektieren und die elektronische Steuerschaltung (12) in einem Eichmodus arbeiten zu lassen, in welchem die Vorrichtung nicht betätigt wird, wenn der Patient durch die Inhalationsleitung (5) einatmet, sondern die elektronische Steuerschaltung den maximalen Unterdruck (ΔPm) bestimmt, der während des Einatmens in der Inhalationsleitung (5) erzeugt wird, und einen Auslöse-Unterdruck (ΔPo) in Abhängigkeit von dem besagten maximalen Unterdruck (ΔPm) berechnet und speichert, wodurch die Vorrichtung im folgenden dann betätigt wird, wenn ein Unterdruck in der Inhalationsleitung (5) detektiert wird, der größer oder gleich dem Auslöse-Unterdruck (ΔPo) ist, und wenn die elektronische Steuerschaltung (12) nicht mehr im Eichmodus arbeitet.

4. Vorrichtung nach Anspruch 2 oder 3, die im übrigen Anzeigemittel (18) umfaßt, die mit der elektronischen Steuerschaltung (12) verbunden sind, um den Wert der vom Patienten eingeatmeten Luftmenge in Abhängigkeit von dem in der Inhalationsleitung (5) gemessenen Unterdruck anzuzeigen.

5. Vorrichtung nach einem der vorhergehenden Ansprüche, die einen Bewegungssensor (17) zum Detektieren einer Bewegung des Vorratbehälters (1) umfaßt, wobei der Bewegungssensor mit der elektronischen Steuerschaltung (12) verbunden und die elektronische Steuerschaltung (12) so ausgebildet ist, daß sie eine Betätigung der Vorrichtung nicht auslöst, wenn der Vorratsbehälter (1) im Verlauf einer zweiten vorbestimmten Zeitdauer (T₂) nicht bewegt worden ist, die der Inhalation durch den Patienten vorausgeht.

6. Vorrichtung nach einem der vorhergehenden Ansprüche, bei der die elektronische Steuerschaltung (12) mit einer Warneinrichtung (18) verbunden und so ausgebildet ist, daß sie die Warneinrichtung (18) ansteuert, um den Patienten zu warnen, daß sich die Vorrichtung nicht in der korrekten Benutzungsposition befindet, wenn der Positionssensor (17) die korrekte Position nicht detektiert.

7. Vorrichtung nach einem der vorhergehenden Ansprüche, bei der die zu zerstäubende Substanz flüssig oder halbflüssig ist, es sich bei der Abgabevorrichtung (2) um eine Pumpe oder ein Aerosolventil handelt, die elektronische Steuerschaltung (12) mit Mitteln (14,68,69) zum Detektieren der Verschiebung des Betätigungsorgans (3) aus seiner Betätigungslage in seine Ruhelage verbunden ist, die elektronische Steuerschaltung (12) mit einer Warnvorrichtung (18) verbunden und die elektronische Steuerschaltung (12) so ausgebildet ist, daß sie die Warnvorrichtung (18) ansteuert, um den Patienten zu warnen, daß die nachfolgende Dosis der besagten Substanz, die von der Vorrichtung abgegeben werden wird, unvollständig sein wird, wenn eine Verschiebung des Betätigungsorgans (3) detektiert wird, während sich die Vorrichtung nicht in ihrer korrekten Ausrichtung befindet.

8. Vorrichtung nach Anspruch 7, die Einrichtungen (71) umfaßt, um von Hand die Pumpe oder das Ventil (2) zu betätigen und bei der die Warneinrichtung (18) dem Patienten anzeigt, die Pumpe oder das Ventil (2) einmal von Hand zu betätigen.

9. Vorrichtung nach einem der Ansprüche 6 bis 8, die Mittel zum Zählen der abgegebenen Dosis-Einheiten der Substanz umfaßt und bei der die Abgabe der nachfolgenden Dosis nicht gezählt wird.

10. Vorrichtung nach einem der vorhergehenden Ansprüche, bei der der Positionssensor (17) eine hohle Säule (17a) umfaßt, die senkrecht ausgerichtet ist, wenn sich die Vorrichtung in ihrer korrekten Position befindet, wobei die hohle Säule (17a) ein unteres Ende (17b) aufweist, das mit zwei elektrischen Kontakten (17c, 17d) versehen ist und die hohle Säule (17a) einen Tropfen Quecksilber (17e) enthält, der die beiden elektrischen Kontakte (17c, 17d) benetzt, wenn sich die Vorrichtung in ihrer korrekten Position befindet.

11. Vorrichtung nach Anspruch 10, bei der der Positionssensor (17) auch als Bewegungssensor dient und die elektronische Steuerschaltung (12) so ausgebildet ist, daß sie die Betätigung der Vorrichtung nicht auslöst, wenn der Vorratsbehälter (1) nicht im Verlauf einer vorbestimmten Zeitdauer (T₂) bewegt worden ist, die der Inhalation durch den Patienten vorausgeht.

12. Vorrichtung nach einem der vorgehenden Ansprüche, bei der die elektrische Betätigungseinrichtung einen Elektromagneten (10) umfaßt, das Verriegelungsorgan (31) mit einem auf Magnetfelder ansprechenden beweglichen Polstück (33) mechanisch verbunden ist, wobei das bewegliche Polstück (33) vor dem Elektromagneten (10) angeordnet ist und der Elektromagnet (10) einen Kern (19) mit permanenter Magnetisierung umfaßt, der das Polstück (33) in einer Warteposition hält, und wobei der Elektromagnet (10) im übrigen eine Wicklung (20) aufweist und die elektronische Steuerschaltung (12) so ausgebildet ist, daß sie die Wicklung (20) mit einem Strom versorgt, der eine Polarisation und Stärke besitzt, die geeignet sind, um das bewegliche Polstück (33) sich in eine Betätigungslage bewegen zu lassen, wobei das bewegliche Polstück (33) das Verriegelungsorgan (31) für eine Bewegung aus seiner Verriegelungsposition in seine Freigabeposition antreibt.

13. Vorrichtung nach Anspruch 12, bei der das bewegliche Polstück (33) wenigstens einen Vorsprung (34) umfaßt, der mit dem Verriegelungsorgan (31) zusammenwirkt, wobei das bewegliche Polstück (33) um eine Achse (51) und das Verriegelungsorgan (31) um eine Achse (49) drehbar montiert ist, wobei die Achsen (49 und 51) mit einer Platte (48) fest verbunden sind, und der Vorsprung (34) das Verriegelungsorgan (31) für eine Drehung aus seiner Verriegelungsstellung, in der es mit einem fest mit dem Vorratsbehälter (1) für das Produkt verbundenen Stift (32) zusammenwirkt, in seine Freigabeposition antreibt.

14. Vorrichtung nach Anspruch 13, bei der der Elektromagnet (10) vierpolig ist und das bewegliche Polstück (33) durch die Opposition identischer Pole in seiner Verriegelungslage in einem instabilen Gleichgewicht gehalten wird, wobei ein Signal der elektronischen Steuerschaltung (12) die Anziehungskraft des Elektromagneten (10) in der Weise vermindert, daß die magnetische Abstoßung, die durch die Opposition der Pole erzeugt wird, das Polstück (33) für eine Drehung in seine Betätigungsposition antreibt.

15. Vorrichtung nach einem der Ansprüche 12 bis 14, bei der ein zweites Verriegelungsorgan (57) vorgesehen ist, das auf einer fest mit der Platine (48) verbundenen Achse (52) drehbar montiert und im wesentlichen mit dem Verriegelungsorgan (31) identisch und bezüglich der Achse (51) zum Verriegelungsorgan (31) symmetrisch angeordnet ist, die das bewegliche Polstück (33) trägt, wobei das zweite Verriegelungsorgan (57) mit einem zweiten Stift (58) zusammenwirkt, der fest mit dem Vorratsbehälter (1) für das Produkt verbunden ist und in seine Freigabeposition durch einen zweiten Vorsprung (59) angetrieben wird, der an dem beweglichen Polstück (33) symmetrisch zum ersten Vorsprung (34) angeordnet ist, wobei das zweite Verriegelungsorgan (57) die Abgabevorrichtung (2) daran hindert, in ihre Ruhelage zurückzukehren.

16. Vorrichtung nach einem der Ansprüche 12 bis 15, bei der das bewegliche Polstück (33) einen Arm (71) umfaßt, der sich für eine von Hand erfolgende Betätigung des beweglichen Polstücks (33) und somit ein Verschieben des Verriegelungsorgans (31) in seine Freigabeposition unabhängig von einem Ansaugvorgang in der Inhalationsleitung über das Gehäuse (21) hinaus erstreckt.

17. Vorrichtung nach einem der vorhergehenden Ansprüche, bei der die Abgabevorrichtung (2) fest mit dem Vorratsbehälter (1) für die zu zerstäubende Substanz verbunden ist, wobei die Betätigungsposition des Betätigungsorgans (3) derart ist, daß das Betätigungsorgan (3) an den Vorratsbehälter (1) angenähert wird, um aus der Ruhelage in die Betätigungslage überzugehen, wobei die tragbare Vorrichtung zum Zerstäuben ein Gehäuse (21) umfaßt, das fest mit der Inhalationsleitung (5) und dem Betätigungsorgan (3) verbunden ist, und wobei die tragbare Zerstäubungsvorrichtung weiterhin eine Kappe (6) aufweist, die am Gehäuse (21) angelenkt und zwischen einer geschlossenen Stellung, in der sie die Inhalationsleitung (5) verschließt, und einer offenen Stellung beweglich ist, in der sie die Inhalationsleitung (5) öffnet, und wobei die tragbare Zerstäubungsvorrichtung weiterhin eine Kompressionsfeder (8) umfaßt, die so ausgebildet ist, daß sie den Vorratsbehälter (1) zum Betätigungsorgan (3) hin vorspannt, um das Betätigungsorgan (3) in seine Betätigungslage zu verschieben, wobei die Kompressionsfeder (8) ein erstes Ende besitzt, das auf den Vorratsbehälter (1) einwirkt, und ein zweites Ende, das auf ein Anlageorgan (7) einwirkt, wobei die Kappe (6) einen einen Hebel (6a) bildenden Teil umfaßt, der am Anlageorgan (7) anliegt, wenn die Kappe (6) in ihre offene Stellung gebracht wird, und dabei die Kompressionsfeder (8) zusammendrückt, und das Verriegelungsorgan (31) den Vorratsbehälter (1) blockiert, wenn es sich in seiner Verriegelungsposition befindet, wobei weiterhin das erste Ende der Kompressionsfeder (8) auf ein Zwischenorgan (22) einwirkt, das am Vorratsbehälter (1) befestigt ist, und das Zwischenorgan (22) einen Stift (32) umfaßt, um das Verriegelungsorgan (31) aufzunehmen, wenn es sich in seiner Verriegelungsposition befindet, so daß das Zwischenorgan (22) mit dem Verriegelungsorgan (31) zusammenwirkt, um den Vorratsbehälter (1) zu blockieren.

18. Vorrichtung nach Anspruch 17, bei der das Betätigungsorgan (3) relativ zur Abgabevorrichtung (2) parallel zu einer Achse (25) verschiebbar ist, bei der die Kappe (6) um zwei Zapfen (26) drehbar montiert ist, und die beiden Zapfen (26) jeweils in einer verlängerten Führungsbahn (27) parallel zur Achse (25) montiert sind und die Zapfen (26) somit axial in ihren Führungsbahnen (27) gegen die Kraft der Kompressionsfeder verschiebbar sind, wobei die Kappe (6) weiterhin zwei seitliche Vorsprünge (28) umfaßt, die nach außen gerichtet sind, und von denen jeder mit einer nach innen gerichteten vorspringenden Oberfläche (29) des Gehäuses zusammenwirkt, um die Zapfen (26) zu zwingen, sich in ihren Führungsbahnen (27) zu verschieben und dabei die Kompressionsfeder (8) zusammenzudrücken, wenn die Kappe (6) aus ihrer geschlossenen in ihre offene Stellung übergeht.

19. Vorrichtung nach Anspruch 18, bei der die beiden Führungsbahnen (27) zwei axiale Enden umfassen und jeweils eine Öffnung (43, 44) aufweisen, die an den beiden Führungsbahnen in der gleichen Höhe an einer Position angeordnet sind, die zwischen den beiden axialen Enden der Führungsbahnen liegt, um das Austreten der Zapfen (26) aus den Führungsbahnen zu ermöglichen, wenn man auf die Kappe (6) parallel zur Achse (25) drückt und gleichzeitig die Kappe (6) senkrecht zur Achse (25) in Richtung der besagten Öffnungen vorspannt, so daß die Kappe (6) auf diese Weise vom Gehäuse (21) demontierbar ist, wobei das Gehäuse (21) eine Füllöffnung (30) mit einer Größe umfaßt, die das Hindurchtreten des Vorratsbehälters (1) der Substanz mit seiner Abgabevorrichtung (2) ermöglicht, wenn die Kappe (6) vom Gehäuse abmontiert ist, wobei das Betätigungsorgan der Abgabevorrichtung (2) in lösbarer Weise am Gehäuse (21) befestigt ist und das Gehäuse Mittel (71) umfaßt, um das Verriegelungsorgan (31) in seine Freigabeposition unabhängig vom Stattfinden eines Ansaugvorganges in der Inhalationsleitung (5) zu verschieben.

20. Vorrichtung nach einem der Ansprüche 17 bis 19, bei der der Teil, der den Hebel (6a) der Kappe (6) bildet, eine Anlageoberfläche (91) in Berührung mit dem Anlageorgan (7) aufweist und die Anlageoberfläche (91) zwei Abflachungen (91a, 91b) umfaßt, die am Anlageorgan (7) in der geschlossenen Position bzw. in der geöffneten Position der Kappe anlegen.

21. Vorrichtung nach einem der Ansprüche 17 bis 20, bei der das Gehäuse (21) eine Öffnung (83) mit einer Größe umfaßt, die ein Hindurchtreten des Vorratsbehälters (1) mit seiner Abgabevorrichtung (2) ermöglicht, um ihn in das Gehäuse (21) einzuführen oder aus dem Gehäuse (21) herauszunehmen, und wobei das Gehäuse (21) weiterhin ein Tragorgan (38) umfaßt, das fest mit dem Betätigungsorgan (3) verbunden und in lösbarer Weise in der Öffnung (83) montiert ist.

22. Vorrichtung nach einem der vorhergehenden Ansprüche, die weiterhin einen Sensor (100) umfaßt, der dazu dient, das Vorhandensein des Vorratsbehälters (1) und/oder der Abgabevorrichtung (2) zu detektieren, wobei der Sensor (100) mit der elektronischen Steuerschaltung (12) verbunden ist, um ein Arbeiten der Vorrichtung zu verhindern, wenn besagter Vorratsbehälter (1) nicht vorhanden ist.

23. Vorrichtung nach einem der vorhergehenden Ansprüche, die im übrigen einen Sensor (100) zum Detektieren des Vorhandenseins des Vorrats (1) und/oder der Abgabevorrichtung (2) umfaßt, wobei der Sensor (100) mit der elektronischen Steuerschaltung (12) verbunden ist, wobei die elektronische Steuerschaltung so ausgebildet ist, daß sie die Anzahl der Vorratsbehälter (1), die in der Vorrichtung positioniert worden sind, in Abhängigkeit von den Signalen zählt, die der Sensor (100) zum Detektieren des Vorhandenseins abgibt.

24. Vorrichtung nach Anspruch 23, bei der die elektronische Steuerschaltung so ausgebildet ist, daß sie jegliche Betätigung der Vorrichtung verhindert, wenn die Anzahl der Vorratsbehälter (1), die in der Vorrichtung positioniert worden sind, über eine vorbestimmte Zahl hinausgeht.
